# EUROPEAN PATENT SPECIFICATION

(11) **EP 3 395 293 B1**
(45) Date of publication and mention of the grant of the patent: **27.05.2020**
(21) Application number: 17168522.5
(22) Date of filing: 27.04.2017
(51) Int. Cl.: A61D 17/00, A61B 5/1455, A61B 5/1464

(54) **FETAL MONITORING SYSTEM**
FÖTUSÜBERWACHUNGSSYSTEM
SYSTÈME DE SURVEILLANCE DU FOETUS

(43) Date of publication of application: 31.10.2018
(73) Proprietor: Veterinärmedizinische Universität Wien, 1210 Vienna (AT)
(72) Inventor: KRIEGER, Stefanie, 2540 Bad Vöslau (AT); KANZ, Peter, 1210 Vienna (AT); DRILLICH, Marc, 2540 Bad Vöslau (AT); IWERSEN, Michael, 2540 Bad Vöslau (AT)
(74) Representative: Sonn & Partner Patentanwälte

(56) References cited:
- WO-A1-2015/197385
- US-A- 5 911 690
- US-A1- 2017 055 496
- US-B1- 6 553 242
- BLEUL ET AL: "Monitoring the bovine fetus during stage II of parturition using pulse oximetry", THERIOGENOLOGY, LOS ALTOS, CA, US, vol. 69, no. 3, 31 October 2007 (2007-10-31), pages 302-311, XP022415237, ISSN: 0093-691X, DOI: 10.1016/J.THERIOGENOLOGY.2007.09.033

## Description

The invention concerns a device and a method for monitoring at least one vital parameter of an animal fetus during parturition. The device comprises a sensor for measuring the at least one vital parameter, including the oxygen saturation of arterial hemoglobin and/or the pulse rate, of the animal fetus, wherein the sensor is arranged inside a hoof cover for being placed on at least one hoof of the animal fetus. The method comprises the steps of attaching a sensor for measuring the at least one vital parameter, including the oxygen saturation of arterial hemoglobin and/or the pulse rate, to at least one hoof of the animal fetus and measuring the at least one vital parameter of the animal fetus using the attached sensor. The device and method are applicable at least to the bovine and/or equine fetus; in particular the device and method are applicable to cattle, horse, sheep, goat and alpaca.

Such devices and methods are widely used for monitoring animal patients in veterinary medicine during intensive care or anesthesiology. Usually they comprise central unit and a sensor unit with a light emitting diode (LED) and a detector diode wherein the central and sensor units are connected via a cable.

It is a well-known problem that calf loss rates are high and have increased in recent years. In the perinatal period, calving problems, in particular dystocia, are the primary causes of mortality. It has been suggested that at a farm-level targeted monitoring should be used to focus on identifiable problems (J. Mee, "Why Do So Many Calves Die on Modern Diary Farms and What Can We Do about Calf Welfare in the Future?," Animals, vol. 3, pp. 1036-1057, 2013).

At present the monitoring of vital parameters of a fetus requires repeated manual intervention, for instance, into the process of parturition. Such intervention can lead to stress of the dam and the fetus and ultimately delay parturition. Furthermore, such manual intervention requires continuous attendance of a person who needs to assess the necessity of momentary examination, which is naturally subjective and error-prone. On the other hand, with omitted or insufficient monitoring of parturition, complications, in particular labor dystocia, may remain undetected leading to an increased risk of stillbirth.

Bleul et al. have performed a study on using pulse oximetry for monitoring the bovine fetus during parturition (U. Bleul and W. Kahn, "Monitoring the bovine fetus during stage II of parturition using pulse oximetry," Theriogenology, vol. 69, pp. 302-311, 2008). They recognized that measurements obtained from the periphery, for instance the legs, are likely to be disturbed by artifacts and therefore unreliable. Consequently they suggest placing an oxygen sensor in the mouth against the mucosa of the hard palate. However, they report that their sensor lost contact in 33% of the cases, again impairing reliability of the monitoring system.

Cabled sensor units like the one used by Bleul et al. require the dam (mother animal) to be fixated during parturition. Such fixation is likely to disturb the process of parturition. Such external disturbances are believed to be part of the problem which leads to the unnaturally high number of stillbirths and calf mortality during parturition.

In a different context, Reix et al. have used a wireless pulse oximeter for studying oxygenation of freely-moving lambs throughout sleep stages and wakefulness (P. Reix et al., "Monitoring pulse oximetry via radiotelemetry in freely-moving lambs", Respiratory Physiology & Neurobiology, vol. 147, pp. 65-72, 2005). Specifically their study is aimed at the analysis of apneas in lambs based on prolonged periods of data collection. They have used sensors attached externally to the root of the tails of the lambs but suggested an implantable version to overcome limitations of the external system. Monitoring vital parameters during parturition is not disclosed by Reix et al. Moreover their device cannot usually be fixed to an animal fetus, because a fixation to the tail of the lamb is hardly possible before parturition is complete. Most of fetuses are in a frontward presentation, a right-side up position, and with both front limbs and head extended into the birth canal. Therefore, during parturition the tail is generally not accessible for fixation of the sensor as disclosed by Reix et al. The device by Reix et al. is therefore neither intended nor suitable for the purpose of the present invention, i.e. monitoring at least one vital parameter of the fetus during parturition.

Uystepruyst et al. have studied the accuracy of oxygen saturation measurements of newborn calves comparing the results of a pulseoximeter and of a blood gas analyser (Ch. Uystepruyst et al. "Evaluation of Accuracy of Pulse Oximetery in Newborn Calves", The Veterinary Journal, vol. 159, pp. 71-76, 2000). Monitoring during parturition is not mentioned. Instead the sensors had been placed on the proximal portion of the tail of the monitored calves. Also no wired or wireless readout of the sensor units is described by Uystepruyst et al. Without a transmission of the measured parameter(s) the sensor is not suitable for the purpose of the present invention because it cannot provide the necessary information to the farmer or veterinarian to allow for monitoring the parturition. Instead, the farmer or veterinarian would need to be present during the entire process of parturition. Moreover, the device by Uystepruyst et al. is not suitable for the purpose of the present invention due to the suggested placement of the sensor on the tail as has been discussed above in connection with Reix et al.

US 2017/0554961 A1 shows a device comprising a sensor for measuring physiological characteristics of an animal, e.g. a temperature sensor, and circuitry to collect information from the temperature sensor and transmit the information to a server.

WO 2015/197385 A1 discloses the use of at least one PPG sensor having at least one light source and at least one photo sensor for detecting vital signs of an animal. The at least one PPG sensor is arranged at least at one position on an animal including at least one of a nose, an ear, a tail, a head or neck, a thigh-bone or leg and a hoof.

US 6,553,242 B1 shows a method and device for measurement of a level of at least one blood constituent. The device and method may be applied to monitoring, inter alia, conditions of apnea, respiratory stress, reduced blood flow in organ regions, heart rate, jaundice, and blood flow velocity.

It is an object of the present invention to further improve on a reduction of stillbirths and calf mortality.

The device according to the present invention is characterized in that the hoof cover is a cast taken from the hoof of an animal of the same species as the animal fetus.

Optionally, the sensor may be connected to a wireless transmitter for wireless transmission of the measured at least one vital parameter. Correspondingly, the method according to the present invention may optionally comprise the step of wirelessly transmitting the at least one vital parameter measured with the sensor.

The wireless transmission is established between the wireless transmitter and a wireless receiver (or receiving unit). Advantageously, the wireless receiver is configured to forward an alarm, e.g. to a cellphone. The use of wireless transmission is particularly advantageous within the present device and method. In addition to the advantage of simplified handling by avoiding cabling that is frequently too long or too short for the application at hand, it allows the parturient mother to move freely while the animal fetus can already be monitored during the second stage of parturition (the stage of expulsion). A fixation or restriction of the dam in a particular position is not necessary and can be avoided. Consequently, the present invention provides means for monitoring at least one vital parameter of the animal fetus during this critical stage while reducing the disturbances of the natural process of parturition compared to the prior art. The wireless transmitter can be battery-powered, in particular it can comprise a rechargeable battery. For example the wireless receiver may be the Root unit provided by Masimo Corporation, USA (www.masimo.com). The wireless receiver may be housed in a protective enclosure, e.g. a plastic box, to protect it from dirt and humidity.

The use of wireless transmission has the further advantage that it easily provides for central collection of data from multiple sensors (and senders) without additional requirements on the receiver. The technology used for wireless transmission may be chosen according to one of the Bluetooth, Wi-Fi or Zigbee standards.

The monitored vital parameters may include one or more of arterial oxygen saturation, pulse rate, perfusion index, Pleth Variability Index, measurements of hemoglobin, carboxyhemoglobin, total oxygen content, methemoglobin, Acoustic Respiration Rate and Pleth Respiration Rate. The monitoring is preferably performed continuously and in real-time in order to achieve early detection of life-threatening situations of the fetus, where intervention of the owner/farmer or the veterinarian is necessary.

Preferably the sensor may include a pulse oximeter, said sensor being preferably a transflectance sensor. This type of sensor allows for continuous and non-invasive monitoring of most or all of the selected vital parameters. The sensor may be an off-the-shelf sensor for capturing vital parameters, e.g. the Radius-7 sensor provided by Masimo Corporation, USA (www.masimo.com). The pulse oximeter may comprise multiple LEDs for emitting light at different wavelengths, which penetrates the adjacent body tissue. Oxygenated and deoxygenated hemoglobin have a different absorption spectrum. The transmitted and reflected (i.e. transflected) residual light can be measured with a detector diode and used to derive the oxygen saturation of the blood.

In a preferred embodiment the pulse oximeter is configured to detect oxygen saturation of arterial hemoglobin below 50%, preferably below 30%.

In the instances discussed above the terms "hoof" and "hoof cover" should be understood as referring generally to an extremity of the fetus and to a corresponding matching cover. More generally the sensor may be arranged inside a toe cover for being placed on at least one toe of the fetus or on a part of a toe of the fetus. This includes covers for being placed on a hoof, a claw or a pad of the fetus. More specifically with regard to hoofs it generally includes covers for solid-hoofed as well as cloven-hoofed animal fetuses. Also it is of no particular importance whether the sensor is attached to a foreleg or a hind leg.

This placement of the sensor achieves a fixation of the sensor in a position where continuous real-time monitoring of a vital parameter of the fetus is possible generally without requiring additional interventions into the parturition. After placing the cover with the sensor (and optionally an external transmitter), the birth and time of labor can continue undisturbed. Specifically, the dam can show the full spectrum of natural behavior during parturition without being hindered by any fixation or other restrictions. Moreover the continuous monitoring eliminates the necessity of subjective and error prone situation-dependent momentary monitoring and provides more objective and reproducible results.

The placement of the sensor at a hoof of the animal fetus can be performed when the hoof passes the pudenal cleft of the dam. As the hooves are usually the first part of the fetus to pass the pudenal cleft for bovine and equine fetuses, monitoring of vital parameters can begin at a relatively early stage of parturition. Consequently a large part of the parturition can be monitored. This placement of the sensor also avoids an intervention within the soft birth canal and reduces the risk of contamination of the birth canal.

Attaching the sensor to at least one hoof of the animal fetus according to the present invention comprises the step of putting a hoof cover on the at least one hoof of the animal fetus, wherein the sensor is arranged inside said hoof cover and the hoof cover is a cast taken from the hoof of an animal of the same species as the animal fetus.

Preferably the hoof cover is made of a resilient, preferably rubber-like material, in particular latex. In particular, the hoof cover may be cast from the hoof of another animal of the same species as the fetus. The inside of the hoof cover essentially matches the shape of the hoof. The material allows the hoof cover to resiliently snuggle to the hoof of the animal fetus once put over it. It provides for rapid and easy attachment of the sensor together with the hoof cover. Thus, both remain firmly attached to the hoof during and after the expulsion of the fetus. Also it can match varying sizes and shapes of the extremity. In one embodiment the sensor may be fixated inside the hoof cover.

In an advantageous embodiment the hoof cover is opaque. In this case the hoof cover protects the sensor from light, which is advantageous when the sensor comprises an optical detector which may be disturbed by ambient light. For example the hoof cover may be made of black latex.

In another preferred embodiment, the sensor may be fixed inside the hoof cover, in particular attached to or integrated with a wall of the hoof cover.

Within the present method, attaching the sensor to at least one hoof of the animal fetus may include attaching the sensor between the hooves of a cloven-hoofed animal fetus. In other words, the sensor may be attached such that it is positioned in the interdigital space of the at least one hoof of the animal fetus to be monitored. This is possible because the hooves of the animal fetus are relatively soft and flexible.

Correspondingly, the hoof cover may be adapted for cloven-hoofed animals and the sensor is arranged centrally inside the hoof cover, such that in use it can be arranged between the hooves of a cloven-hoofed animal.

The wireless transmitter may be mounted to the hoof cover. In this case the wireless transmitter can be attached to the animal fetus together with the sensor, thus avoiding separate parts connected by a cable. This arrangement is particularly advantageous at the final stage of parturition when the animal fetus separates from the dam, because monitoring can be continued without displacement of the transmitter. Preferably the sensor and the wireless transmitter are integrated in a single combined transmission and sensor unit.

More specifically the wireless transmitter may be connected to the sensor inside the hoof cover by a wire.

After attaching the sensor to the animal fetus, a wireless transmitter may be attached to the parturient mother animal. Specifically the wireless transmitter may be attached to the tail of the dam. Specifically it may be attached in the proximal third of the length of the tail of the dam. It has turned out particularly useful to apply a bandage around the tail and the wireless transmitter to hold it in place, in particular a flexible, cohesive bandage, e.g. a PetFlex bandage by Andover Healthcare, Inc. Alternatively or additionally, a clamp or comparable device may be used for fixation of the wireless transmitter. The transmitter when fixed to the dam naturally remains in close vicinity of the animal fetus and the sensor attached to it. The transmitter may be larger than the sensor alone. Attaching it to the mother is easier and more reliable while avoiding pollution and injuries of the fetus.

The present method may include: monitoring the wirelessly transmitted at least one vital parameter and triggering an alarm when the at least one vital parameter is outside a pre-defined range for at least a pre-defined duration.

Specifically the present method may require monitoring a wirelessly transmitted oxygen saturation and triggering an alarm when the oxygen saturation is below a pre-defined threshold for at least a pre-defined duration. For example, the pre-defined threshold may be 30 % or 50 % and the pre-defined duration may be two minutes.

Alternatively or additionally, the present method may require monitoring a wirelessly transmitted pulse rate and triggering an alarm when the pulse and/or heart rate is outside a pre-defined range. For example, the pre-defined range may be 80-120 contractions of the heart per minute (bpm).

In order to facilitate uninterrupted monitoring, it is advantageous to provide the present invention in a set for monitoring at least one vital parameter of an animal fetus during parturition, the set comprising a device as defined above and a computer program product comprising program parts, which when loaded onto a computer are designed to receive at least one vital parameter from the device, monitor the at least one vital parameter and trigger an alarm when the at least one vital parameter is outside a pre-defined range for at least a pre-defined duration.

In the following, preferred embodiments of the method, the apparatus and the seed product according to the invention will be defined, as well as preferred combinations thereof:
1. Device for monitoring at least one vital parameter of an animal fetus during parturition, the device comprising a sensor for measuring the at least one vital parameter, including the oxygen saturation of arterial hemoglobin and/or the pulse rate, of the animal fetus, wherein the sensor is arranged inside a hoof cover for being placed on at least one hoof of the animal fetus, characterized in that the hoof cover is a cast taken from the hoof of an animal of the same species as the animal fetus.
2. Device according to embodiment 1, characterized in that the sensor includes a pulse oximeter, said sensor being preferably a transflectance sensor.
3. Device according to embodiment 2, characterized in that the pulse oximeter is configured to detect oxygen saturations of arterial hemoglobin below 50%, preferably below 30%.
4. Device according to embodiment 1 or 2, characterized in that the sensor is connected to a wireless transmitter for wireless transmission of the measured at least one vital parameter.
5. Device according to one of embodiments 1 to 4, characterized in that the hoof cover is made of a resilient, preferably rubber-like material, in particular latex.
6. Device according to one of embodiments 1 to 5, characterized in that the hoof cover is opaque.
7. Device according to one of embodiments 1 to 6, characterized in that the sensor is fixed inside the hoof cover, in particular attached to or integrated with a wall of the hoof cover.
8. Device according to one of embodiments 1 to 7, characterized in that the hoof cover is adapted for cloven-hoofed animals and the sensor is arranged centrally inside the hoof cover, such that in use it can be arranged between the hooves of a cloven-hoofed animal.
9. Device according to embodiment 4, characterized in that the wireless transmitter is mounted to the hoof cover.
10. Device according to embodiment 4, characterized in that the wireless transmitter is connected to the sensor inside the hoof cover by a wire.
11. Method for monitoring at least one vital parameter of an animal fetus during parturition, the method comprising the steps of:
   - attaching a sensor for measuring the at least one vital parameter, including the oxygen saturation of arterial hemoglobin and/or the pulse rate, to at least one hoof of the animal fetus;
   - measuring the at least one vital parameter of the animal fetus using the attached sensor;
   characterized in that attaching the sensor to at least one hoof of the animal fetus comprises the step of putting a hoof cover on the at least one hoof of the animal fetus, wherein the sensor is arranged inside said hoof cover and the hoof cover is a cast taken from the hoof of an animal of the same species as the animal fetus.
12. Method according to embodiment 11, characterized by
   - wirelessly transmitting the at least one vital parameter measured with the sensor.
13. Method according to embodiment 11 or 12, characterized by attaching the sensor between the hooves of a cloven-hoofed animal fetus.
14. Method according to one of embodiments 11 to 13, characterized in that after attaching the sensor to the animal fetus, a wireless transmitter is attached to the parturient mother animal.
15. Method according to one of embodiments 11 to 14, characterized by monitoring the wirelessly transmitted at least one vital parameter and triggering an alarm when the at least one vital parameter is outside a pre-defined range for at least a pre-defined duration.
16. Method according to embodiment 15, characterized by monitoring a wirelessly transmitted oxygen saturation and triggering an alarm when the oxygen saturation is below a pre-defined threshold for at least a pre-defined duration.
17. Method according to embodiment 15 or 16, characterized by monitoring a wirelessly transmitted pulse rate and triggering an alarm when the pulse and/or heart rate is outside a pre-defined range.
18. Set for monitoring at least one vital parameter of an animal fetus during parturition, the set comprising a device according to one of embodiment 1 to 10 and a computer program product comprising program parts, which when loaded onto a computer are designed to receive at least one vital parameter from the device, monitor the at least one vital parameter and trigger an alarm when the at least one vital parameter is outside a pre-defined range for at least a pre-defined duration.

The invention will be defined in more detail below by means of preferred exemplary embodiments, to which it is not to be limited to, however, and with reference to the drawings. In detail:
Fig. 1 shows a device comprising a hoof cover with an integrated sensor and wireless transmitter;
Fig. 2 schematically shows a device comprising a hoof cover with an integrated sensor connected with an external transmitter.
Fig. 3 shows the fixation of a wireless transmitter at the tail of a dam during parturition;
Fig. 4 shows a different device, which is not an embodiment of the present invention, for application to a nose of the animal fetus.

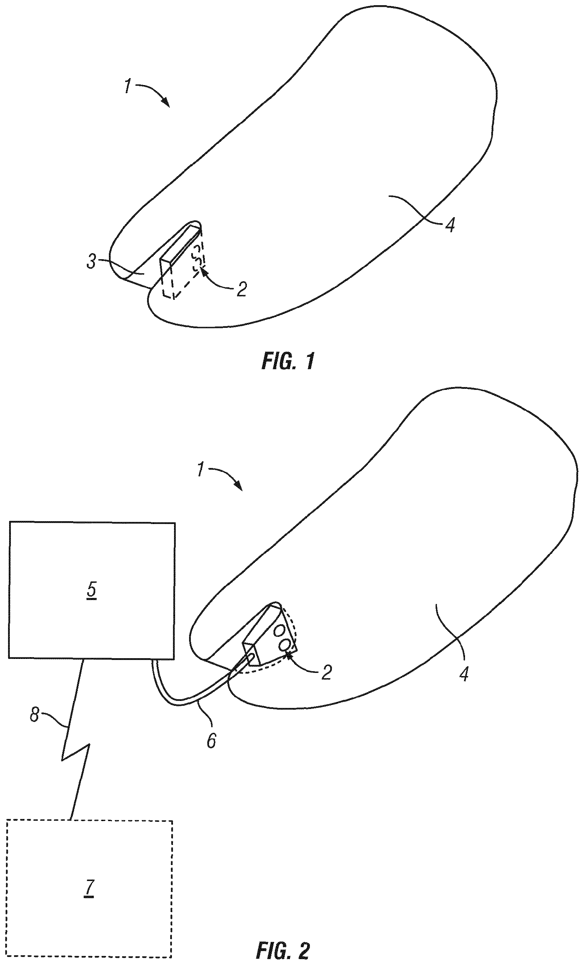

Fig. 1 shows a device 1 for monitoring vital parameters of a bovine fetus during parturition. The device comprises a sensor 2 for measuring vital parameters of the fetus. Specifically the sensor 2 is configured for monitoring the oxygen saturation of arterial hemoglobin and the pulse rate of the fetus. The sensor 2 is integrated with a wall 3 of a hoof cover 4 and thereby arranged and fixed inside the hoof cover 4 for being placed on at least one hoof of the bovine fetus.

The hoof cover 4 is a cast taken from the hoof of a calf. It is made of black latex and has a wall thickness sufficient for rendering the cover opaque to ambient light. In this embodiment the wall thickness is between 0.5 and 2 mm, in particular between 0.5 and 1 mm or approximately 1 mm. The wall of the hoof cover 4 with which the sensor 2 is integrated is arranged centrally such as to separate the hooves of the cloven-hoofed animal, i.e. in an interdigital position between the two hooves.

The sensor 2 includes a pulse oximeter. More specifically the sensor is a transflectance sensor. The pulse oximeter is configured to detect oxygen saturation of arterial hemoglobin below 30%. In the embodiment shown in Fig. 1, the sensor 2 is connected to an integrated wireless transmitter, i.e. the sensor and wireless transmitter are integrated in a common housing mounted to and fixed in the hoof cover 4. The wireless transmitter is configured for wireless transmission of the measured vital parameters.

Fig. 2 shows an alternative embodiment of the device according to the present invention. The hoof cover 4 and sensor 2 are generally the same as discussed above in connection with the embodiment shown in Fig. 1. In contrast to Fig. 1, the wireless transmitter 5 of the embodiment shown in Fig. 2 is an external wireless transmitter connected to the sensor 2 inside the hoof cover 4 by a wire 6.

As indicated in Fig. 2, the wireless transmitter 5 may be connected with a wireless receiver 7 via a wireless link 8 (the same holds for the embodiment of Fig. 1). The wireless link 8 may be established according to one of the Bluetooth, Wi-Fi or Zigbee standards.

In order to use one of the devices 1 shown in Fig. 1 or Fig. 2 for monitoring the oxygen saturation of arterial hemoglobin and the pulse rate of an animal fetus during parturition, the sensor 2 for measuring said vital parameters is first attached to an extremity of the animal fetus once it passes the pudenal cleft of the dam.

In the present example the fetus is cloven-hoofed, i.e. the device 1 shown in Fig. 1 or 2 comprises a hoof cover 4 suitable for use with a cloven-hoofed fetus. The hoof cover 4 is attached to the fetus by putting the hoof cover over the hoof of the fetus. At the same time the sensor 2, which is arranged inside the hoof cover 4, is attached between the two hooves of one extremity, which is accessible earliest during the stage of expulsion. After the sensor 2 is in place and attached to the animal fetus, an optional wireless transmitter 5 (cf. Fig. 2) can be separately attached to the dam (the parturient mother animal) .

After the sensor 2 and optionally the wireless transmitter 5 are attached, the vital parameters of the animal fetus are continuously measured using the attached sensor 2. The measured vital parameters are wirelessly transmitted to a wireless receiver 7 as indicated in Fig. 2. The wireless receiver 7 monitors the wirelessly transmitted vital parameters. In particular it verifies for each wirelessly transmitted and received measurement of the oxygen saturation whether it is below a pre-defined threshold, e.g. 30 %, for at least a pre-defined duration, e.g. two minutes. Similarly, it verifies for each wirelessly transmitted and received measurement of the pulse and/or heart rate whether it is outside a pre-defined range, e.g. outside the range between 80 and 120 bpm. When it observes that at least one vital parameter is outside its pre-defined range(s) for at least a pre-defined duration, it triggers an alarm in order to raise the attention of the farmer or veterinarian supervising the parturition.

The receiving and monitoring functions explained above may be implemented as a computer program product, which can run on a generic wireless receiver capable of receiving standards-compliant wireless transmission signals and emitting or triggering an alarm. For example, the wireless receiver may be a smartphone or a tablet computer. It is within the scope of the present invention that a device 1 according to one of the embodiments shown in Fig. 1 or 2 and described above can be provided in a set together with such a computer program product, wherein the computer program product may be delivered e.g. on a computer readable medium or as an access reference, a download link or an access code to unlock a copy of the program downloaded from a third party.

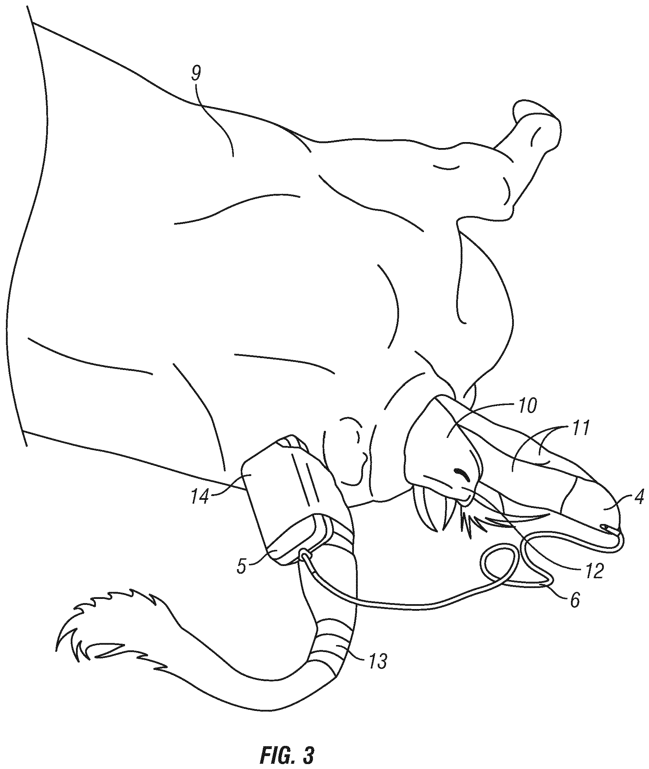

Fig. 3 shows the rear part 9 of a dam during parturition, when expulsion of the fetus 10 has begun. The forelegs 11 and nose 12 of the fetus 10 have already left the birth canal and are visible. A hoof cover 4 according to Fig. 1 and 2 is attached to the hooves at one of the forelegs 11. The hoof cover 4 is connected to a wireless transmitter 5 by a wire 6. The wireless transmitter 5 is attached to the tail 13 at a proximal portion of the tail 13. The attachment is achieved by a PetFlex bandage 14 wrapped around the wireless transmitter 5 and the tail 13.

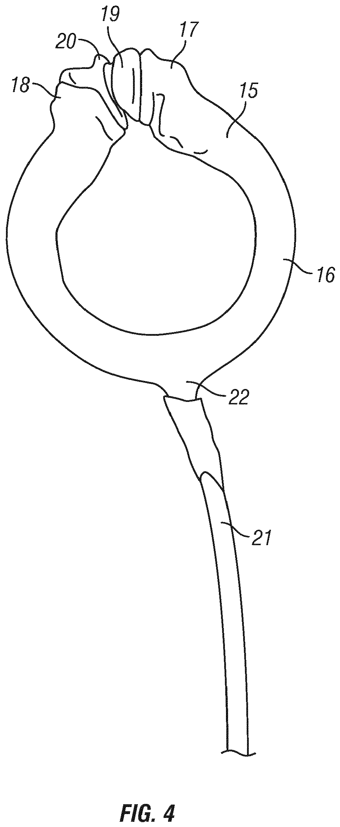

Fig. 4 shows a different device 15 for application to a nose of the animal fetus. The device 15 comprises a roughly horseshoeshaped resilient clip 16, wherein the two ends 17, 18 of the clip 16 are arranged closely together. A transflectance sensor 19 is arranged at one end 17 of the clip 16 and a reflective surface 20 facing the transflectance sensor 19 is arranged at the other end 18 of the clip 16. The surface of the transflectance sensor 19 is convex and the reflective surface 20 is concave. The transflectance sensor 19 is connected by a cable 21 extending from the center 22 of the bend of the clip 16 with a wireless transmitter (not shown). In order to attach the device 15 to the animal fetus, the septum of the nose is received between the two ends 17, 18 of the clip 16. It is held at the nose by means of the restoring force of the clip 16, which forces the two ends 17, 18 of the clip 16 against each other and against the two sides of the septum.

## Claims

1. Device (1) for monitoring at least one vital parameter of an animal fetus during parturition, the device (1) comprising a sensor (2) for measuring the at least one vital parameter, including the oxygen saturation of arterial hemoglobin and/or the pulse rate, of the animal fetus, wherein the sensor (2) is arranged inside a hoof cover (4) for being placed on at least one hoof of the animal fetus, **characterized in that** the hoof cover (4) is a cast taken from the hoof of an animal of the same species as the animal fetus.

2. Device (1) according to claim 1, **characterized in that** the sensor (2) includes a pulse oximeter, said sensor (2) being preferably a transflectance sensor.

3. Device (1) according to claim 1 or 2, **characterized in that** the sensor (2) is connected to a wireless transmitter (5) for wireless transmission of the measured at least one vital parameter.

4. Device (1) according to one of claims 1 to 3, **characterized in that** the hoof cover (4) is made of a resilient, preferably rubber-like material, in particular latex.

5. Device (1) according to one of claims 1 to 4, **characterized in that** the hoof cover (4) is opaque.

6. Device (1) according to one of claims 1 to 5, **characterized in that** the sensor (2) is fixed inside the hoof cover (4), in particular attached to or integrated with a wall (3) of the hoof cover (4).

7. Device (1) according to one of claims 1 to 6, **characterized in that** the hoof cover (4) is adapted for cloven-hoofed animals and the sensor (2) is arranged centrally inside the hoof cover (4), such that in use it can be arranged between the hooves of a cloven-hoofed animal.

8. Method for monitoring at least one vital parameter of an animal fetus during parturition, the method comprising the steps of:
- attaching a sensor (2) for measuring the at least one vital parameter, including the oxygen saturation of arterial hemoglobin and/or the pulse rate, to at least one hoof of the animal fetus;
- measuring the at least one vital parameter of the animal fetus using the attached sensor (2);
**characterized in that** attaching the sensor (2) to at least one hoof of the animal fetus comprises the step of putting a hoof cover (4) on the at least one hoof of the animal fetus, wherein the sensor (2) is arranged inside said hoof cover (4) and the hoof cover (4) is a cast taken from the hoof of an animal of the same species as the animal fetus.

9. Method according to claim 8, **characterized by**:
- wirelessly transmitting the at least one vital parameter measured with the sensor (2).

10. Method according to claim 8 or 9, **characterized by** attaching the sensor (2) between the hooves of a cloven-hoofed animal fetus.

11. Method according to one of claims 8 to 10, **characterized in that** after attaching the sensor (2) to the animal fetus, a wireless transmitter (5) is attached to the parturient mother animal.

12. Set for monitoring at least one vital parameter of an animal fetus during parturition, the set comprising a device (1) according to one of claims 1 to 7 and a computer program product comprising program parts, which when loaded onto a computer are designed to receive at least one vital parameter from the device (1), monitor the at least one vital parameter and trigger an alarm when the at least one vital parameter is outside a pre-defined range for at least a pre-defined duration.

## Patentansprüche

1. Vorrichtung (1) zur Überwachung mindestens eines Vitalparameters eines Tierfötus im Verlauf des Geburtsvorgangs, wobei die Vorrichtung (1) einen Sensor (2) zum Messen des mindestens einen Vitalparameters des Tierfötus umfasst, einschließend die Sauerstoffsättigung von arteriellem Hämoglobin und/oder die Pulsfrequenz, wobei der Sensor (2) im Inneren eines Huf- oder Klauenschuhs (4) angeordnet ist, der zum Anlegen an mindestens einen Huf oder mindestens eine Klaue des Tierfötus vorgesehen ist, **dadurch gekennzeichnet, dass** es sich bei dem Huf- oder Klauenschuh (4) um eine Abformung von einem Huf oder einer Klaue eines Tiers der gleichen Spezies wie der des betreffenden Tierfötus handelt.

2. Vorrichtung (1) nach Anspruch 1, **dadurch gekennzeichnet, dass** der Sensor (2) ein Pulsoximeter aufweist, wobei es sich bei dem Sensor (2) bevorzugt um einen Transflexionssensor handelt.

3. Vorrichtung (1) nach Anspruch 1 oder 2, **dadurch gekennzeichnet, dass** der Sensor (2) zum Zweck der drahtlosen Übertragung des mindestens einen gemessenen Vitalparameters mit einer zur drahtlosen Übertragung vorgesehenen Vorrichtung (5) verbunden ist.

4. Vorrichtung (1) nach einem der Ansprüche 1 bis 3, **dadurch gekennzeichnet, dass** der Huf- oder Klauenschuh (4) aus einem resilienten, bevorzugt gummiartigen, Material gefertigt ist, insbesondere aus Latex.

5. Vorrichtung (1) nach einem der Ansprüche 1 bis 4, **dadurch gekennzeichnet, dass** der Huf- oder Klauenschuh (4) opak ist.

6. Vorrichtung (1) nach einem der Ansprüche 1 bis 5, **dadurch gekennzeichnet, dass** der Sensor (2) im Inneren des Huf- oder Klauenschuhs (4) fixiert, insbesondere an einer Wand (3) des Huf- oder Klauenschuhs (4) befestigt oder in diese integriert ist.

7. Vorrichtung (1) nach einem der Ansprüche 1 bis 6, **dadurch gekennzeichnet, dass** der Huf- oder Klauenschuh (4) für paarhufige Tiere vorgesehen ist und der Sensor (2) zentral im Inneren des Huf- oder Klauenschuhs (4) angeordnet ist, so dass der Sensor (2) im Gebrauch zwischen den Klauen eines paarhufigen Tiers angeordnet werden kann.

8. Verfahren zur Überwachung mindestens eines Vitalparameters eines Tierfötus im Verlauf des Geburtsvorgangs, wobei das Verfahren die folgenden Schritte umfasst:
- das an mindestens einen Huf oder mindestens eine Klaue des Tierfötus erfolgende Anbringen eines Sensors (2) zum Messen des mindestens einen Vitalparameters, einschließend die Sauerstoffsättigung von arteriellem Hämoglobin und/oder die Pulsfrequenz;
- das Messen des mindestens einen Vitalparameters des Tierfötus unter Verwendung des angebrachten Sensors (2);
**dadurch gekennzeichnet, dass** das an mindestens einen Huf oder mindestens eine Klaue des Tierfötus erfolgende Anbringen des Sensors (2) den Schritt des Anlegens eines Huf- oder Klauenschuhs (4) an den mindestens einen Huf oder die mindestens eine Klaue des Tierfötus umfasst, wobei der Sensor (2) im Inneren des Huf- oder Klauenschuhs (4) angeordnet ist und es sich bei dem Huf- oder Klauenschuh (4) um eine Abformung von einem Huf oder einer Klaue eines Tiers der gleichen Spezies wie der des betreffenden Tierfötus handelt.

9. Verfahren nach Anspruch 8, **gekennzeichnet durch**:
- das drahtlose Übertragen des mit dem Sensor (2) gemessenen mindestens einen Vitalparameters.

10. Verfahren nach Anspruch 8 oder 9, **dadurch gekennzeichnet, dass** der Sensor (2) zwischen den Klauen eines paarhufigen Tierfötus angebracht wird.

11. Verfahren nach einem der Ansprüche 8 bis 10, **dadurch gekennzeichnet, dass** im Anschluss an das an den Tierfötus erfolgende Anbringen des Sensors (2) eine zur drahtlosen Übertragung vorgesehene Vorrichtung (5) an dem gebärenden Muttertier angebracht wird.

12. Set zur Überwachung mindestens eines Vitalparameters eines Tierfötus im Verlauf des Geburtsvorgangs, wobei das Set umfasst: eine Vorrichtung (1) nach einem der Ansprüche 1 bis 7 sowie ein Computerprogrammprodukt, das Programmkomponenten umfasst, die nach dem Laden auf einen Computer dazu ausgelegt sind, mindestens einen Vitalparameter von der Vorrichtung (1) zu empfangen, den mindestens einen Vitalparameter zu überwachen und einen Alarm auszulösen, wenn der mindestens eine Vitalparameter für eine vorbestimmte Mindestzeitdauer außerhalb eines vorbestimmten Bereichs liegt.

## Revendications

1. Dispositif (1) pour surveiller au moins un paramètre vital d'un fœtus animal pendant la naissance, le dispositif (1) comprenant un capteur (2) pour mesurer le au moins un paramètre vital, comprenant la saturation en oxygène de l'hémoglobine artérielle et/ou le pouls du fœtus animal, dans lequel le capteur (2) est agencé à l'intérieur d'un couvercle de sabot (4) pour être placé sur au moins un sabot du fœtus animal, **caractérisé en ce que** le couvercle de sabot (4) est une empreinte prélevée sur le sabot d'un animal de la même espèce que le fœtus animal.

2. Dispositif (1) selon la revendication 1, **caractérisé en ce que** le capteur (2) comprend un oxymètre de pouls, ledit capteur (2) étant de préférence un capteur de transflectance.

3. Dispositif (1) selon la revendication 1 ou 2, **caractérisé en ce que** le capteur (2) est raccordé à un émetteur sans fil (5) pour la transmission sans fil du au moins un paramètre vital mesuré.

4. Dispositif (1) selon l'une des revendications 1 à 3, **caractérisé en ce que** le couvercle de sabot (4) est réalisé avec un matériau résilient, de préférence du type caoutchouc, en particulier du latex.

5. Dispositif (1) selon l'une des revendications 1 à 4, **caractérisé en ce que** le couvercle de sabot (4) est opaque.

6. Dispositif (1) selon l'une des revendications 1 à 5, **caractérisé en ce que** le capteur (2) est fixé à l'intérieur du couvercle de sabot (4), en particulier fixé sur ou intégré avec une paroi (3) du couvercle de sabot (4).

7. Dispositif (1) selon l'une des revendications 1 à 6, **caractérisé en ce que** le couvercle de sabot (4) est adapté pour les animaux biongulés et le capteur (2) est agencé au centre dans le couvercle de sabot (4), de sorte qu'à l'usage, il peut être agencé entre les sabots d'un animal biongulé.

8. Méthode pour surveiller au moins un paramètre vital d'un fœtus animal pendant la naissance, la méthode comprenant les étapes consistant à :
- fixer un capteur (2) pour mesurer le au moins un paramètre vital, comprenant la saturation en oxygène d'hémoglobine artérielle et/ou le pouls, sur au moins un sabot du fœtus animal ;
- mesurer le au moins un paramètre vital du fœtus animal à l'aide du capteur (2) fixé ;
**caractérisée en ce que** l'étape consistant à fixer le capteur (2) sur au moins un sabot du fœtus animal comprend l'étape consistant à placer un couvercle de sabot (4) sur le au moins un sabot du fœtus animal, dans laquelle le capteur (2) est agencé à l'intérieur dudit couvercle de sabot (4) et le couvercle de sabot (4) est une empreinte prélevée sur le sabot d'un animal de la même espèce que le fœtus animal.

9. Méthode selon la revendication 8, **caractérisée par** l'étape consistant à :
- transmettre sans fil le au moins un paramètre vital mesuré par le capteur (2).

10. Méthode selon la revendication 8 ou 9, **caractérisée par** l'étape consistant à fixer le capteur (2) entre les sabots d'un fœtus animal biongulé.

11. Méthode selon l'une des revendications 8 à 10, **caractérisée en ce qu'**après l'étape consistant à fixer le capteur (2) sur le fœtus animal, un émetteur sans fil (5) est fixé sur la femelle parturiente.

12. Ensemble pour surveiller au moins un paramètre vital d'un fœtus animal pendant la naissance, l'ensemble comprenant un dispositif (1) selon l'une quelconque des revendications 1 à 7 et un produit de programme informatique comprenant des parties de programme qui, lorsqu'elles sont chargées sur un ordinateur, sont conçues pour recevoir au moins un paramètre vital du dispositif (1), surveiller le au moins un paramètre vital et déclencher une alarme lorsque le au moins un paramètre vital n'est plus dans une plage prédéfinie pendant au moins une durée prédéfinie.
